# EUROPEAN PATENT APPLICATION

(11) **EP 2 767 542 A1**
(43) Date of publication of application: **20.08.2014**
(21) Application number: 13155593.0
(22) Date of filing: 18.02.2013
(51) Int. Cl.: C07F 15/02, C08F 4/54, C08F 10/02

(54) **Oligomerization catalyst**

(71) Applicant: Basell Polyolefine GmbH, 50389 Wesseling (DE)
(72) Inventor: Schuhen, Katrin, 68167 Mannheim (DE); Mihan, Shahram, 65812 Bad Soden (DE); Fantinel, Fabiana, 37122 Verona (IT)
(74) Representative: Sacco, Marco

(57) **Abstract**

A late transition metal compound of formula (I) 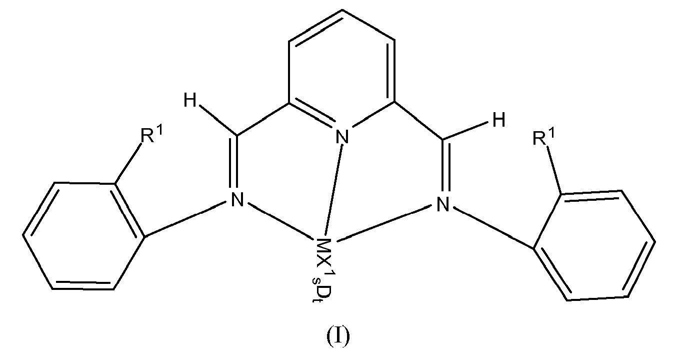 Wherein
M is Fe[II], Fe[III], Co[I], Co[II], Co[III], Ru[II], Ru[III], Ru[IV], Mn[I], Mn[II], Mn[III] or Mn[IV];
the radicals X¹, equal to or different from each other, are hydrogen atoms, halogen atoms, R, OR, OSO₂CF₃, OCOR, SR, NR₂ or PR₂ groups, wherein R is a linear or branched, saturated or unsaturated C₁-C₂₀ alkyl, C₃-C₂₀ cycloalkyl, C₆-C₂₀ aryl, C₇-C₂₀ alkylaryl or C₇-C₂₀ arylalkyl radical, optionally containing heteroatoms belonging to groups 13-17 of the Periodic Table of the Elements; or two X¹ can optionally form a substituted or unsubstituted butadienyl radical or a OR'O group wherein R' is a divalent radical selected from C₁-C₂₀ alkylidene, C₆-C₄₀ arylidene, C₇-C₄₀ alkylarylidene and C₇-C₄₀ arylalkylidene radicals;
D is an uncharged donor; s is 1, 2, 3 or 4, t ranges from 0 to 4, preferably t is 0, 1 or 2.

## Description

The present invention relates to new catalyst systems and to their use in the oligomerization of alpha-olefins; said catalyst systems are particularly useful in ethylene oligomerization in order to obtain α-olefinic oligomers having from 4 to 24 carbon atoms.

High linear α-olefins, i.e. alpha-olefins having from 4 to 24 carbon atoms, and more specifically 6 to 20 carbon atoms, are in great demand as intermediates in the preparation of detergents, lubricant additives and polyolefins; alpha-olefins having 6-20 carbon atoms are suitable for a large number of applications in different technical fields.

However, oligomerization processes commonly known in the state of the art lead to the obtainment, together with the target products, of undesirable by-products, such as internal olefins, branched olefins and olefins having a number of carbon atoms outside the above mentioned range.

Catalyst system able to give oligomers are known in the art, for example The international patent application WO 96/27439 describes a new class of oligomerization catalysts comprising a bridged bis-amido Group 4 metal compound in association with suitable activating agents.

From another point of view catalyst system based on late transition metal catalyst having a tridentate ligand are known in the art to give ethylene polymers. For example WO 04/074333 describes among others 2,6-bis(1-(2,6-diisopropylphenylamido)ethylidene) pyridine complexes of yttrium, lanthanide and actinide metals as catalysts for the polymerization of conjugated dienes.

WO 1999/12981 relates to a broad class of late transition metal complexes having a tridentate ligand. Some of these complexes results to give oligomer material, however this material has not been analized and furthermore the polymerization activity are quite low The applicant found a catalyst system based on a late transition metal compound able to give oligomers without the production of by products having higher molecular weight. An object of the present invention is therefore a late transition metal compound of formula (I) 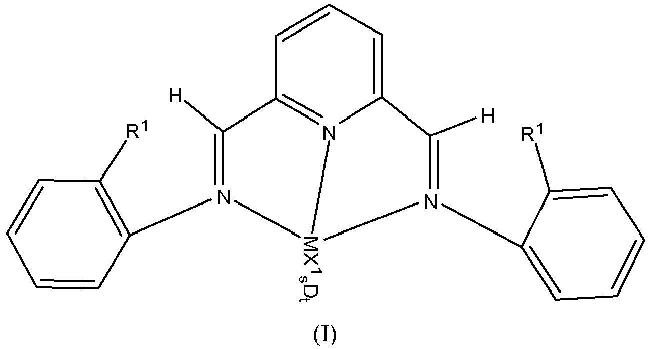 Wherein
M is Fe[II], Fe[III], Co[I], Co[II], Co[III], Ru[II], Ru[III], Ru[IV], Mn[I], Mn[II], Mn[III] or Mn[IV]; preferably M is Fe[II], Fe[III]; more preferably M is Fe[II];
the radicals X¹, equal to or different from each other, are hydrogen atoms, halogen atoms, R, OR, OSO₂CF₃, OCOR, SR, NR₂ or PR₂ groups, wherein R is a linear or branched, saturated or unsaturated C₁-C₂₀ alkyl, C₃-C₂₀ cycloalkyl, C₆-C₂₀ aryl, C₇-C₂₀ alkylaryl or C₇-C₂₀ arylalkyl radical, optionally containing heteroatoms belonging to groups 13-17 of the Periodic
Table of the Elements; or two X¹ can optionally form a substituted or unsubstituted butadienyl radical or a OR'O group wherein R' is a divalent radical selected from C₁-C₂₀ alkylidene, C₆-C₄₀ arylidene, C₇-C₄₀ alkylarylidene and C₇-C₄₀ arylalkylidene radicals; preferably X¹ is a hydrogen atom, a halogen atom or a R group; more preferably X is chlorine or a methyl radical;

D is an uncharged donor; s is 1, 2, 3 or 4, preferably s is 2 or 3; t ranges from 0 to 4, preferably t is 0, 1 or 2.

R¹, equal to or different from each other, are C₁-C₁₀ hydrocarbon radicals optionally containing heteroatoms belonging to groups 13-17 of the Periodic Table of the Elements; preferably R¹ are linear or branched, cyclic or acyclic, C₁-C₁₀-alkyl, C₂-C₁₀ alkenyl, C₂-C₁₀ alkynyl radicals optionally containing heteroatoms belonging to groups 13-17 of the Periodic Table of the Elements; more preferably R¹ are C₁-C₈ alkyl radicals such as methyl, ethyl isopropyl radicals;

The class of compounds of formula (I) are able to give, when used in ethylene polymerization reaction, oligomers having low number of carbon atoms without the presence of waxes or ethylene polymers.

Thus a further object of the present invention is a catalyst system obtainable by contacting:
A) a compound of formula (I) as above described;
B) an alumoxane or a compound capable of forming an alkyl cation with the compound of formula (I).

The alumoxanes used in the catalyst system according to the invention are considered to be linear, branched or cyclic compounds containing at least one group of the type: 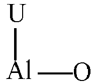 wherein the substituents U, same or different, are defined above.

In particular, alumoxanes of the formula: 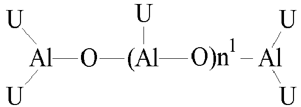 can be used in the case of linear compounds, wherein n¹ is 0 or an integer of from 1 to 40 and the substituents U are defined as above; or alumoxanes of the formula: 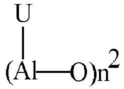 can be used in the case of cyclic compounds, wherein n² is an integer from 2 to 40 and the U substituents are defined as above.

Examples of alumoxanes suitable for use according to the present invention are methylalumoxane (MAO), tetra-(isobutyl)alumoxane (TIBAO), tetra-(2,4,4-trimethylpentyl)alumoxane (TIOAO), tetra-(2,3-dimethylbutyl)alumoxane (TDMBAO) and tetra-(2,3,3-trimethylbutyl)alumoxane (TTMBAO).

Particularly interesting cocatalysts are those described in WO 99/21899 and in WO01/21674 in which the alkyl and aryl groups have specific branched patterns.

Non-limiting examples of aluminium compounds that can be reacted with water to give suitable alumoxanes (b), described in WO 99/21899 and WO01/21674, are: tris(2,3,3-trimethyl-butyl)aluminium, tris(2,3-dimethyl-hexyl)aluminium, tris(2,3-dimethylbutyl)aluminium, tris(2,3-dimethyl-pentyl)aluminium, tris(2,3-dimethyl-heptyl)aluminium, tris(2-methyl-3-ethyl-pentyl)aluminium, tris(2-methyl-3-ethyl-hexyl)aluminium, tris(2-methyl-3-ethyl-heptyl)aluminium, tris(2-methyl-3-propyl-hexyl)aluminium, tris(2-ethyl-3-methylbutyl)aluminium, tris(2-ethyl-3-methyl-pentyl)aluminium, tris(2,3-diethyl-pentyl)aluminium, tris(2-propyl-3-methyl-butyl)aluminium, tris(2-isopropyl-3-methyl-butyl)aluminium, tris(2-isobutyl-3-methyl-pentyl)aluminium, tris(2,3,3-trimethyl-pentyl)aluminium, tris(2,3,3-trimethyl-hexyl)aluminium, tris(2-ethyl-3,3-dimethyl-butyl)aluminium, tris(2-ethyl-3,3-dimethyl-pentyl)aluminium, tris(2-isopropyl-3,3-dimethyl-butyl)aluminium, tris(2-trimethylsilyl-propyl)aluminium, tris(2-methyl-3-phenyl-butyl)aluminium, tris(2-ethyl-3-phenyl-butyl)aluminium, tris(2,3-dimethyl-3-phenyl-butyl)aluminium, tris(2-phenylpropyl)aluminium, tris[2-(4-fluoro-phenyl)-propyl]aluminium, tris[2-(4-chloro-phenyl)-propyl]aluminium, tris[2-(3-isopropyl-phenyl)-propyl]aluminium, tris(2-phenylbutyl)aluminium, tris(3-methyl-2-phenyl-butyl)aluminium, tris(2-phenyl-pentyl)aluminium, tris[2-(pentafluorophenyl)-propyl]aluminium, tris[2,2-diphenyl-ethyl]aluminium and tris[2-phenyl-2-methyl-propyl]aluminium, as well as the corresponding compounds wherein one of the hydrocarbyl groups is replaced with a hydrogen atom, and those wherein one or two of the hydrocarbyl groups are replaced with an isobutyl group.

Amongst the above aluminium compounds, trimethylaluminium (TMA), triisobutylaluminium (TIBA), tris(2,4,4-trimethyl-pentyl)aluminium (TIOA), tris(2,3-dimethylbutyl)aluminium (TDMBA) and tris(2,3,3-trimethylbutyl)aluminium (TTMBA) are preferred.

Non-limiting examples of compounds able to form an alkylmetallocene cation are compounds of formula D⁺E⁻, wherein D⁺ is a Brønsted acid, able to donate a proton and to react irreversibly with a substituent X of the metallocene of formula (I) and E⁻ is a compatible anion, which is able to stabilize the active catalytic species originating from the reaction of the two compounds, and which is sufficiently labile to be removed by an olefinic monomer. Preferably, the anion E⁻ comprises one or more boron atoms. More preferably, the anion E⁻ is an anion of the formula BAr₄⁽⁻⁾, wherein the substituents Ar which can be identical or different are aryl radicals such as phenyl, pentafluorophenyl or bis(trifluoromethyl)phenyl. Tetrakispentafluorophenyl borate is particularly preferred compound, as described in WO 91/02012. Moreover, compounds of formula BAr₃ can be conveniently used. Compounds of this type are described, for example, in the International patent application WO 92/00333. Other examples of compounds able to form an alkylmetallocene cation are compounds of formula BAr₃P wherein P is a substituted or unsubstituted pyrrol radical. These compounds are described in WO01/62764. Compounds containing boron atoms can be conveniently supported according to the description of DE-A-19962814 and DE-A-19962910. All these compounds containing boron atoms can be used in a molar ratio between boron and the metal of the metallocene comprised between about 1:1 and about 10:1; preferably 1:1 and 2.1; more preferably about 1:1.

Non limiting examples of compounds of formula D⁺E⁻ are:
Tributylammoniumtetra(pentafluorophenyl)aluminate,
Tributylammoniumtetra(trifluoromethylphenyl)borate,
Tributylammoniumtetra(4-fluorophenyl)borate,
N,N-Dimethylbenzylammonium-tetrakispentafluorophenylborate,
N,N-Dimethylhexylamonium-tetrakispentafluorophenylborate,
N,N-Dimethylaniliniumtetrakis(pentafluorophenyl)borate,
N,N-Dimethylaniliniumtetrakis(pentafluorophenyl)aluminate,
N,N-Dimethylbenzylammonium-tetrakispentafluorophenylborate,
N,N-Dimethylhexylamonium-tetrakispentafluorophenylborate,
Di(propyl)ammoniumtetrakis(pentafluorophenyl)borate,
Di(cyclohexyl)ammoniumtetrakis(pentafluorophenyl)borate,
Triphenylcarbeniumtetrakis(pentafluorophenyl)borate,
Triphenylcarbeniumtetrakis(pentafluorophenyl)aluminate,
Ferroceniumtetrakis(pentafluorophenyl)borate,
Ferroceniumtetrakis(pentafluorophenyl)aluminate.
Triphenylcarbeniumtetrakis(pentafluorophenyl)borate, and
N,N-Dimethylaniliniumtetrakis(pentafluorophenyl)borate.

The catalysts system of the present invention can be supported on an inert support. This is achieved by depositing component A) or the product of the reaction thereof with the component B), or the component B) and then component A) on an inert support. The support can be a porous solid such as talc, a sheet silicate, an inorganic oxide or a finely divided polymer powder (e.g. polyolefin). Suitable inorganic oxides may be found among the oxides of elements of groups 2, 3, 4, 5, 13, 14, 15 and 16 of the Periodic Table of the Elements. Examples of oxides preferred as supports include silicon dioxide, aluminum oxide, and also mixed oxides of the elements calcium, aluminum, silicon, magnesium or titanium and also corresponding oxide mixtures, magnesium halides, styrene/divinylbenzene copolymers, polyethylene or polypropylene. Other inorganic oxides which can be used alone or in combination with the abovementioned preferred oxidic supports are, for example, MgO, ZrO₂, TiO₂ or B₂O₃.

A suitable class of supports which can be used is that constituted by porous organic supports functionalized with groups having active hydrogen atoms. Particularly suitable are those in which the organic support is a partially crosslinked styrene polymer. Supports of this type are described in European application EP-633 272.

Another class of inert supports particularly suitable for use according to the invention is that of polyolefin porous prepolymers, particularly polyethylene.

The support materials used preferably have a specific surface area in the range from 10 to 1000m²/g, a pore volume in the range from 0.1 to 5ml/g and a mean particle size of from 1 to 500µm. Preference is given to supports having a specific surface area in the range from 50 to 500m²/g, a pore volume in the range from 0.5 to 3.5ml/g and a mean particle size in the range from 5 to 350µm. Particular preference is given to supports having a specific surface area in the range from 200 to 400m²/g, a pore volume in the range from 0.8 to 3.0 ml/g and a mean particle size of from 10 to 300µm.

The inorganic support can be subjected to a thermal treatment, e.g. to remove adsorbed water. Such a drying treatment is generally carried out at from 80 to 300°C, preferably from 100 to 200°C, with drying at from 100 to 200°C preferably being carried out under reduced pressure and/or a blanket of inert gas (e.g. nitrogen), or the inorganic support can be calcined at from 200 to 1000°C to produce the desired structure of the solid and/or set the desired OH concentration on the surface. The support can also be treated chemically using customary desiccants such as metal alkyls, preferably aluminum alkyls, chlorosilanes or SiCl₄, or else methylaluminoxane. Appropriate treatment methods are described, for example, in WO 00/31090.

The inorganic support material can also be chemically modified. For example, treatment of silica gel with (NH₄)₂SiF₆ leads to fluorination of the silica gel surface, or treatment of silica gels with silanes containing nitrogen-, fluorine- or sulfur-containing groups leads to correspondingly modified silica gel surfaces.

Organic support materials such as finely divided polyolefin powders (e.g. polyethylene, polypropylene or polystyrene) can also be used and are preferably likewise freed of adhering moisture, solvent residues or other impurities by means of appropriate purification and drying operations before use. It is also possible to use functionalized polymer supports, e.g. supports based on polystyrene, via whose functional groups, for example ammonium or hydroxy groups, at least one of the catalyst components can be immobilized. The solid compound obtained by supporting the catalyst system object of the present invention on a carrier in combination with the further addition of the alkylaluminium compound either as such or prereacted with water if necessary, can be usefully.

Preferred support is silica.

Thus a further object of the present invention is a supported catalyst system obtainable by contacting:
A) a compound of formula (I) as above described;
B) an alumoxane or a compound capable of forming an alkyl cation with the compound of formula (I); and
C) an inert support.

With the catalyst system of the present invention it is possible to obtain hydrocarbon oligomers having a carbon number from 4 to 20 preferably from 4 to 16, more preferably from 4 to 12.

Thus a further object of the present invention is an oligomerization process of ethylene comprising the step of contacting ethylene under polymerization conditions in the presence of the catalyst system described above.

The polymerization process can be carried out using all industrially known polymerization methods at temperatures in the range from -60 to 350°C, preferably from 0 to 200°C and particularly preferably from 25 to 150°C, and under pressures of from 0.5 to 4000 bar, preferably from 1 to 100 bar and particularly preferably from 3 to 40 bar. The polymerization can be carried out in a known manner in bulk, in suspension, in the gas phase or in a supercritical medium in the customary reactors used for the polymerization of olefins. It can be carried out batchwise or preferably continuously in one or more stages. High-pressure polymerization processes in tube reactors or autoclaves, solution processes, suspension processes, stirred gas-phase processes and gas-phase fluidized-bed processes are all possible.

The polymerizations are usually carried out at temperatures in the range from -60 to 350°C, preferably in the range from 20 to 300°C, and under pressures of from 0.5 to 4000 bar. The mean residence times are usually from 0.5 to 5 hours, preferably from 0.5 to 3 hours. The advantageous pressure and temperature ranges for carrying out the polymerizations usually depend on the polymerization method. In the case of high-pressure polymerization processes, which are customarily carried out at pressures of from 1000 to 4000 bar, in particular from 2000 to 3500 bar, high polymerization temperatures are generally also set. Advantageous temperature ranges for these high-pressure polymerization processes are from 200 to 320°C, in particular from 220 to 290°C. In the case of low-pressure polymerization processes, it is usual to set a temperature which is at least a few degrees below the softening temperature of the polymer. In particular, temperatures of from 50 to 180°C, preferably from 70 to 120°C, are set in these polymerization processes. In the case of suspension polymerizations, the polymerization is usually carried out in a suspension medium, preferably an inert hydrocarbon such as isobutane or mixtures of hydrocarbons or else in the monomers themselves. The polymerization temperatures are generally in the range from -20 to 115°C, and the pressure is generally in the range from 1 to 100bar. The polymerization can be carried out either batchwise, e.g. in stirring autoclaves, or continuously, e.g. in tube reactors, preferably in loop reactors. Particular preference is given to employing the Phillips PF process as described in US 3,242,150 and US 3,248,179. The gas-phase polymerization is generally carried out in the range from 30 to 125°C at pressures of from 1 to 50bar.

Among the abovementioned polymerization processes, particular preference is given to gas-phase polymerization, in particular in gas-phase fluidized-bed reactors, solution polymerization and suspension polymerization, in particular in loop reactors and stirred tank reactors. The gas-phase polymerization can also be carried out in the condensed or supercondensed mode, in which part of the circulating gas is cooled to below the dew point and is recirculated as a two-phase mixture to the reactor. Furthermore, it is possible to use a multizone reactor in which the two polymerization zones are linked to one another and the polymer is passed alternately through these two zones a number of times. The two zones can also have different polymerization conditions. Such a reactor is described, for example, in WO 97/04015. The different or identical polymerization processes can also, if desired, be connected in series so as to form a polymerization cascade, for example as in the Hostalen® process.

The polymerization is preferably carried out in a single reactor, in particular in a gas-phase reactor.

The following examples are given for illustrative purposes and do not intend to limit the scope of the invention.

### Examples

### Sysnthesis of 2,6-bis[(2-methyl phenylimino)]pyridine iron (II) dichloride (A-1) (compound according to the invention)

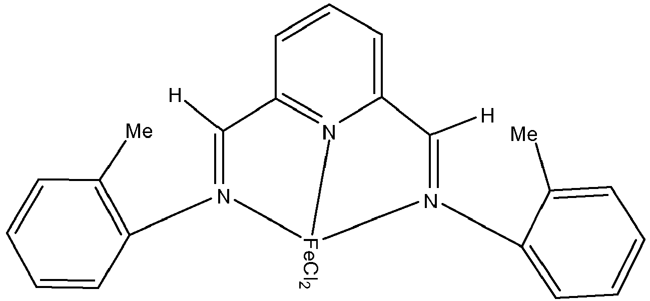

2,6-bis [(2-methyl phenylimino)]pyridine iron (II) dichloride has been prepared according to the procedure of example 10 of WO 99/12981 excepting that 2- methyl aniline has been used instead of 2,6-dimethylaniline.

### Sysnthesis of 2,6-bis[1-(2-methyl phenylimino)-methyl]pyridine iron (II) dichloride (C-1) (comparative compound)

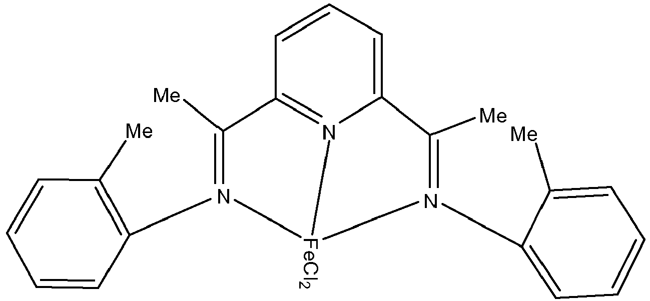

2,6-bis[1-(2-methyl phenylimino)-methyl]pyridine iron (II) dichloride has been synthesized according to example 5 of WO 99/12981.

### Preparation of the catalyst system

10 kg of spray-dried silica Sylopol® XP02326 obtained from Grace GmbH & Co. KG, Worms, Germany were calcinated at 600°C for 6 hours and then stored at 10°C. 4.64 mmol g of compound A-1 or C-1 was dissolved in a mixture of 66.1 mL of toluene and of 93.5 mL of a 30% wt._% solution of MAO in toluene obtained from Chemtura Organometallics GmbH, Bergkamen, Germany. The solution was added to 98.7 g of the pre-treated spray-dried silica at 0°C. A wet powder, not free-flowing, was obtained. Afterward the catalyst was stirred for two hours at 10°C. Thereafter, the catalyst was suspended in 500 mL of heptane. The suspension was filtered over an argon overlaid frit and the obtained solid was dried under argon flow. 187.5 g of a powder of reed green color were obtained.

The theoretical loading was 50 µmol of catalyst component/g of silica and the theoretical molar ratio of Al:Fe was 90:1.

### Ethylene oligomerization example 1 and comparative example 2

A 1 1 Steel autoclave was flushed with Argon at 70°C. Then 150 mg of Triisobutylaluminium (TIBA in heptane 50 mg/ml) as well as 50 mg Costelan AS 100 (Costelan in heptane 50mg/ml) were added.
80 mg of the catalyst obtained as described above was then added, by a lance to avoid contact with air. Ethylene pressure was adjusted to 10 bar. The total pressure of 26 bar at 70°C was kept constant for 1 hour via adding additional ethylene fed in constant ratio to ethylene variable from 0 to 0.1 ml/g, during the oligomerization. After one hour the pressure was released and the oligomers (+ waxes in comparative examples) were removed from the autoclave. The resulting product was analyzed, the results are shown in table 1

| Oligomer | Ex 1 | Comparative Ex 2 | Oligomer | Ex 1 | Comparative Ex 2 |
|---|---|---|---|---|---|
| C4 % | 2.71 | 4.63 | C20 % | 0 | 13.02 |
| C6% | 42.70 | 0 | C22 % | 0 | 10.85 |
| C8 % | 46.53 | 0 | C24 % | 0 | 8.63 |
| C10 % | 7.64 | 0 | C26 % | 0 | 7.78 |
| C12 % | 0.35 | 3.45 | C28 % | 0 | 6.68 |
| C14 % | 0 | 9.66 | C30 % | 0 | 6.12 |
| C16 % | 0 | 11.46 | C32 % | 0 | 4.86 |
| C18 % | 0 | 12.66 | Polymer g | 0 | 4.2 |

From table 1 the improved distribution of comonomers obtained by the oligormerizaiton catalyst of the present invention clearly results.

## Claims

1. A late transition metal compound of formula (I) 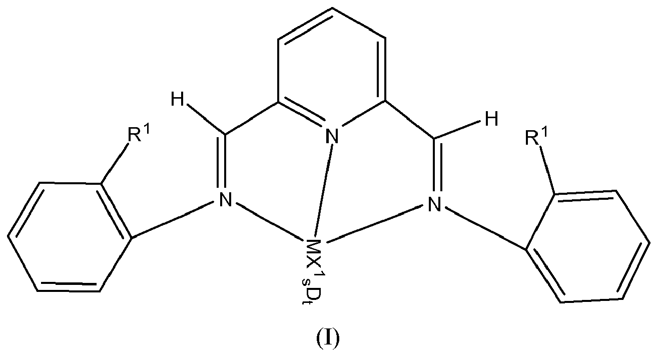 Wherein
M is Fe[II], Fe[III], Co[I], Co[II], Co[III], Ru[II], Ru[III], Ru[IV], Mn[I], Mn[II], Mn[III] or Mn[IV];
the radicals X¹, equal to or different from each other, are hydrogen atoms, halogen atoms, R, OR, OSO₂CF₃, OCOR, SR, NR₂ or PR₂ groups, wherein R is a linear or branched, saturated or unsaturated C₁-C₂₀ alkyl, C₃-C₂₀ cycloalkyl, C₆-C₂₀ aryl, C₇-C₂₀ alkylaryl or C₇-C₂₀ arylalkyl radical, optionally containing heteroatoms belonging to groups 13-17 of the Periodic Table of the Elements; or two X¹ can optionally form a substituted or unsubstituted butadienyl radical or a OR'O group wherein R' is a divalent radical selected from C₁-C₂₀ alkylidene, C₆-C₄₀ arylidene, C₇-C₄₀ alkylarylidene and C₇-C₄₀ arylalkylidene radicals;
D is an uncharged donor; s is 1, 2, 3 or 4, t ranges from 0 to 4, preferably t is 0, 1 or 2. R¹, equal to or different from each other, are C₁-C₁₀ hydrocarbon radicals optionally containing heteroatoms belonging to groups 13-17 of the Periodic Table of the Elements;

2. The late transition metal compound of formula (I) according to claim 1 wherein: M is Fe[II] or Fe[III].

3. The late transition metal compound of formula (I) according to claims 1 or 2 wherein X¹ is a hydrogen atom, a halogen atom or a R group wherein R is a linear or branched, saturated or unsaturated C₁-C₂₀ alkyl, C₃-C₂₀ cycloalkyl, C₆-C₂₀ aryl, C₇-C₂₀ alkylaryl or C₇-C₂₀ arylalkyl radical, optionally containing heteroatoms belonging to groups 13-17 of the Periodic Table of the Elements;

4. The late transition metal compound of formula (I) according to anyone of claims 1-3 wherein R¹ is a C₁-C₈ alkyl radicals; s is 2 or 3 and t is 0, 1 or 2.

5. A catalyst system obtainable by contacting:
A) a compound of formula (I) as described in claims 1-4;
B) an alumoxane or a compound capable of forming an alkyl cation with the compound of formula (I).

6. The catalyst system according to claim 5 further containing:
C) an inert support.

7. The catalyst system according to claim 6 wherein the inert support is silica.

8. An oligomerization process of ethylene comprising the step of contacting ethylene under polymerization conditions in the presence of the catalyst system of claims 1.7.

9. The oligomerization process according to claim 8 carried out in a gas phase.
